Europäisches Patentamt

European Patent Office

Office européen des brevets

(19)

(11) Veröffentlichungsnummer: **0 486 917 A2**

## (12) EUROPÄISCHE PATENTANMELDUNG

(21) Anmeldenummer: **91119188.0**

(22) Anmeldetag: **11.11.91**

(51) Int. Cl.5: **C12N 15/35**, C12P 21/02,
A61K 35/76, G01N 33/569,
C12Q 1/70, C07K 13/00

(30) Priorität: **17.11.90 DE 4036784**

(43) Veröffentlichungstag der Anmeldung:
**27.05.92 Patentblatt 92/22**

(84) Benannte Vertragsstaaten:
**AT BE CH DE DK ES FR GB GR IT LI LU NL SE**

(71) Anmelder: **BEHRINGWERKE
Aktiengesellschaft
Postfach 1140
W-3550 Marburg 1(DE)**

(72) Erfinder: **Rittner, Karola
Märzgasse 3
W-6900 Heidelberg(DE)**
Erfinder: **Sczakiel, Georg, Dr.
Goethestrasse 36
W-6915 Dossenheim(DE)**

(74) Vertreter: **Aulmich, Gerhard, Dr. et al
Hoechst AG Zentrale Patentabteilung
Postfach 80 03 20
W-6230 Frankturt am Main 80(DE)**

(54) **Antivirale Aktivität des vom adeno-assoziierten Virus Typ 2 codierten rep-Gens.**

(57) Die Erfindung betrifft die Verwendung eines aus dem adeno-assoziierten Virus Typ 2 stammenden rep-Gens mit antiviraler Aktivität gegenüber dem HIV-1 Virus.

EP 0 486 917 A2

Diese Erfindung betrifft die Verwendung des durch das adenoassoziierte Virus Typ 2 (AAV-2) codierten rep-Gens für die Herstellung eines spezifischen antiviralen Agens.

Weiterhin betrifft die Erfindung Plasmide mit antiviraler Aktivität und einen diagnostischen Kit.

Das menschliche Immun-Mangelsyndrom (AIDS) ist in den vergangenen Jahren zu einem ernsten Problem für die menschliche Gesellschaft geworden. Obwohl es einige Anmeldungen gibt, die sich mit der Bekämpfung des humanen Immundefizienzvirus (HIV), das mit der AIDS-Erkrankung (kausal) assoziiert ist, befassen, ist eine Bekämpfung dieses Virus bis heute nur ansatzweise gelungen. Einige Mittel sind in der Lage, eine Ausbreitung des Virus in vivo zu reduzieren und erhöhen damit die Lebenserwartung der befallenen Patienten. Jedoch wurde bis heute ein Medikament oder eine Behandlung, die das Virus vollständig zerstört und damit die Gesundheit einer befallenen Person wiederherstellt, nicht gefunden. Alle bis heute verwendeten Arzneimittel gegen HIV besitzen einen schwerwiegenden Nachteil: sie haben sehr starke Nebenwirkungen.

Die Erfinder waren sich dieser großen Nachteile bei der Behandlung von HIV bewußt und haben deshalb nach alternativen antiviralen Faktoren gesucht, die die ernsten Nebeneffekte bekannter Medikamente nicht mehr zeigen sollten.

Hemmung der Replikation und der Potenz für eine Transformation des Adenovirus (5) und des Herpes simplex-Virus (6) durch den adeno-assoziierten Virus (AAV) sowie auch die Inhibierung der zellulären Transformation, die durch das Rinder-Papillomavirus unterstützt wird (Virology, 172, S. 253-261 (1989)) und die Inhibierung der von HSV induzierten Genamplifikation (Journal of Virology, 64, S. 3012-3018 (1990)) wurden ebenfalls beobachtet.

Das AAV ist ein Helfer-Virus-abhängiges Parvovirus mit einem einzelsträngigen DNA-Genom mit einer ungefähren Länge von 5 kb (Advances in Virus Research, 32, S 243-307 (1987)).

Das Virus hat einen weiten Wirtsbereich und ist abhängig von Helferfunktionen anderer Viren. Jedoch scheint dieses Virus nicht abhängig von Gewebefaktoren oder artspezifischen zellulären Faktoren zu sein. Das Genom enthält zwei nicht-überlappende offene Leseraster, wobei das 3'-gelegene drei virale Mantelproteine codiert und das zweite, das "rep" genannt wird, für vier bekannte rep-Proteine mit Molukargewichten von 78 kd, 68 kd, 52 kd und 40 kd codiert (Journal of Virology 60, s. 823-832 (1986)). Für das 68 kd rep-Protein wurde eine DNA-bindende Kapazität sowie eine ATP-abhängige Endonuklease-Aktivität und eine DNA-Helikase-Aktivität nachgewiesen (Cell 61, S. 447-457 (1990)). Es ist bekannt, daß die Produkte des offenen Leserasters (rep) die eigenen Promotoren p5 und p19 in trans regulieren (J. Virol. 63, S. 4450-4454 (1989); Mol. Cel. Biol. 6, S. 2884-2894 (1986)).

Die Erfinder untersuchten eine mögliche negative Interferenz, die sich bei der Replikation des HIV Typ 1 in Gegenwart von intakter AAV-2 DNA ergibt.

Das Ziel der vorliegenden Erfindung ist es, Faktoren bereitzustellen, die eine antivirale Aktivität besitzen. Ein weiteres Ziel der vorliegenden Erfindung ist es, speziell einen Faktor zu finden, der HIV-1 inhibierende Aktivität besitzt.

Das Ziel der Erfindung ist durch den Gegenstand des Anspruchs 1 erfüllt. Die Erfinder haben gefunden, daß das sogenannte rep-Gen, das durch das adeno-assoziierte Virus Typ 2 (AAV-2) codiert wird, eine antivirale Aktivität besitzt. Damit kann das rep-Gen oder eine Teilsequenz des Gens für die Herstellung eines spezifischen antiviralen Faktors verwendet werden.

Insbesondere kann mit dem rep-Gen oder Teilsequenzen dieses Gens das HIV-Virus Typ 1 bekämpft werden.

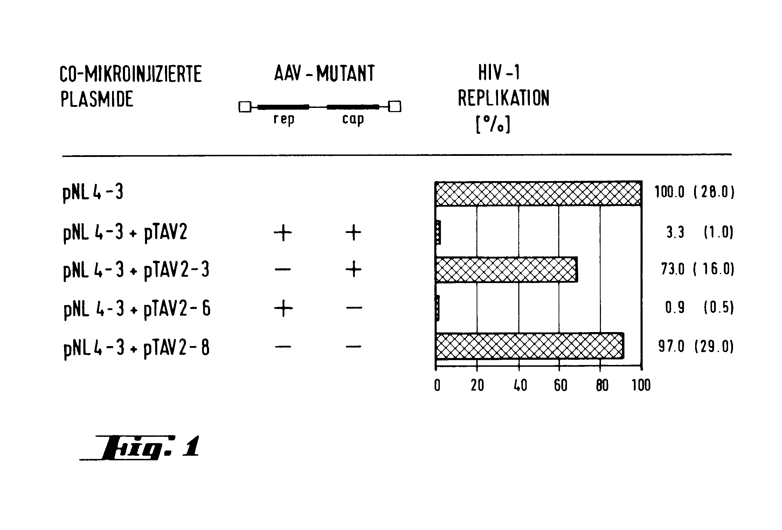

Die Effekte des AAV rep offenen Leserasters, das im kompletten AAV-Genom integriert ist, auf die HIV-1 Replikation wurden in einem transienten Testsystem untersucht (Biochem. Biophys. Res. Comm. 169, S. 643-651 (1990)). Dieses System basiert auf der Co-Mikroinjektion des AAV-Wildtyps oder einer mutanten DNA (J. Virol. 64, S. 3012-3018 (1990)), zusammen mit einer infektiösen HIV-1 proviralen DNA (Klon pNL4-3) (J. Virol. 59, S. 284-291 (1986)) in die Kerne von humanen Epitheloid-SW 480-Zellen. Diese Zellen erlauben die HIV-1 Replikation nach Transfektion, können jedoch nicht mit HIV-1 Virus infiziert werden (J. Virol. 59, S. 284-291 (1986)). HIV-1 Virus wurde anfänglich in co-mikroinjizierten Zellen hergestellt und wurde vermehrt durch Co-Kultivierung mit humanen T-lymphoiden MT-4 Zellen. HIV-1 wurde danach in einem zellfreien Co-Kultur-Überstand mit Hilfe eines kommerziellen HIV-1 Antigen ELISA gemessen (Biochem. Biophys. Res. Comm. 169, S. 643-651 (1990)). Aus diesen Ergebnissen, die in Fig. 1 zusammengefaßt wurden, kann man ersehen, daß die starke Hemmung einer HIV-1 Replikation bei einem molaren Verhältnis von HIV-1 DNA und AAV-DNA von 1 : 10 in anfänglich co-mikroinjizierten Zellen korreliert ist mit der Anwesenheit eines intakten AAV rep offenen Leserasters. Die in der Fig. 1 erwähnten Plasmide (pTAV) wurden beschrieben (Heilbronn et al 1990; J. Virology 64: 3012-3018 bzw. AAV-2 Wildtyp-Sequenz in Laughlin et al. (1983), Gene 23: 65-73). Eine Inaktivierung des rep offenen Leserasters, bei der das cap offene Leseraster gleichzeitig intakt bleibt, erlaubt eine völlig normale HIV-1 Replikation. Eine Hemmung bei inaktiviertem rep offenen Leseraster ist nicht zu beobachten. Weiterhin ist zu beobachten, daß die rep$^+$, rep$^-$ Mutante (pTAV2-3), die nicht in der Lage sein sollte, AAV Viruspartikel zu produzieren - selbst dann nicht, wenn eine hypothetische Helfer-Funktion des HIV-1 Virus für die AAV Replikation in SW480-Zellen angenommen werden kann - dieselbe anti-HIV-1-Wirksamkeit besitzt wie der AAV Wildtyp (rep$^+$, cap$^+$). Diese Beobachtung macht deutlich, daß die Hemmung der HIV-1 Replikation tatsächlich in den mikroinjizierten SW480-Zellen stattfindet und daß dieser Effekt keineswegs durch einen Artefakt hervorgerufen wird, der in einem späten Stadium der Co-Kultur auftritt. Die Produkte der Translation des AAV rep offenen Leserasters sind an der Hemmung der HIV-1 Replikation beteiligt. Deshalb ist ein weiteres bevorzugtes Element der Erfindung, daß die Polypeptide, die nach Translation des offenen Leserasters des AAV rep Gens entstehen, für die Herstellung eines spezifischen antiviralen Faktors verwendet werden können. Diese Proteine können auch Bestandteil eines diagnostischen Kits sein, der wenigstens eines der vier Proteine enthält, die nach der Translation des offenen Leserasters des AAV rep Gens erhalten werden.

Die rep-Gen-vermittelte Hemmung der HIV-1 Replikation ist kein Ergebnis einer möglichen Zelltoxizität der rep-Proteine und basiert deshalb auch nicht auf der Unfähigkeit der befallenen Zellen zur HIV-1 Replikation durch Zerstörung des zelleigenen Syntheseapparats. Die Co-Mikroinjektion eines Rous-Sarcoma Virus LTR-getriebenen β-Galactosidase-Gens mit rep$^+$ (pTAV2) oder rep$^-$ - (pTAV2-3) DNA führt zu keiner Differenz im Prozentsatz von mikroinjizierten Zellen mit β-Galactosidase-Genexpression, wenn diese Expression 24 h nach Injektion gemessen wird. Dies zeigt deutlich, daß SW480-Zellen sehr wohl eine Genexpression in der Gegenwart des rep-Gens erlauben und daß konsequenterweise die mikroinjizierten Zellen ihre Potenz zur HIV-1 Genexpression und zur Virusherstellung behalten.

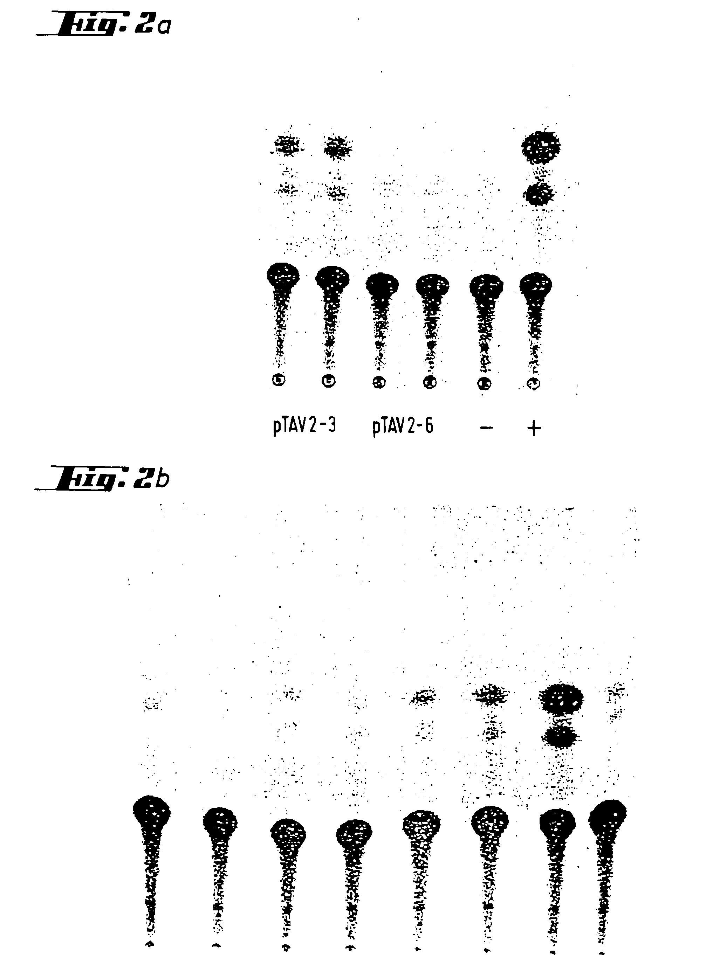

Die AAV-abhängige Hemmung der HIV-1 Virusreplikation ist von einem intakten rep-Gen abhängig. Es bleibt jedoch offen, welche Elemente des HIV-1 Virus an der negativen Interferenz beteiligt sind. Um zu analysieren, ob Sequenzen, die vom HIV-1 5'-LTR an der repabhängigen Hemmung der HIV-1 Replikation beteiligt sind, wurde die HIV-1 LTR (U3/R-Anteil) getriebene Expression der Chloramphenicolacetyltransferase (CAT) als ein Indikatorgen beobachtet. Das CAT Expressionsniveau wurde in der Gegenwart eines intakten AAV rep-Gens gemessen, wobei der oben beschriebene Test verwendet wurde. Die Ergebnisse zeigen, daß pTAV2-6 (rep$^+$), aber nicht pTAV2-3 (rep$^-$) die CAT-Expression (Fig. 2a) in einer dosisabhängigen Weise (Fig. 2b) hemmt. Dies bedeutet, daß der U3/R-Anteil des HIV-1 5'-LTR für eine rep-abhängige Hemmung der Genexpression genügt. Jedoch schließt dies nicht aus, daß das rep-Gen auch noch weitere Schritte der HIV-1 Replikation beeinflußt.

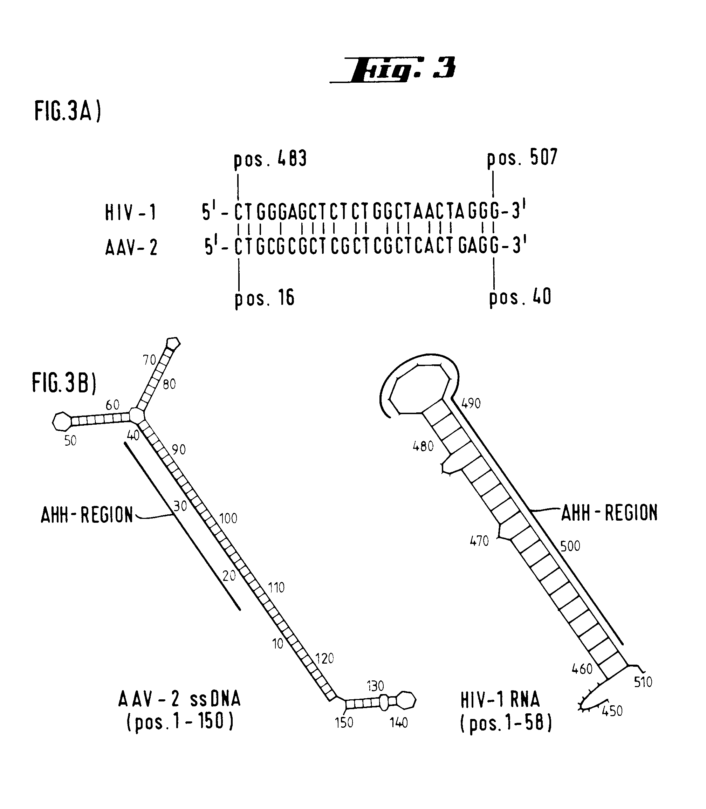

Die Nukleotidpositionen sind für HIV-1 nach Adachi et al. (1986) J. Virol., 59, 284-291 und für AAV-2 nach Berns und Bohenzky (1987) Adv. Virus Res., 32, 243-307 (Fig. 3).

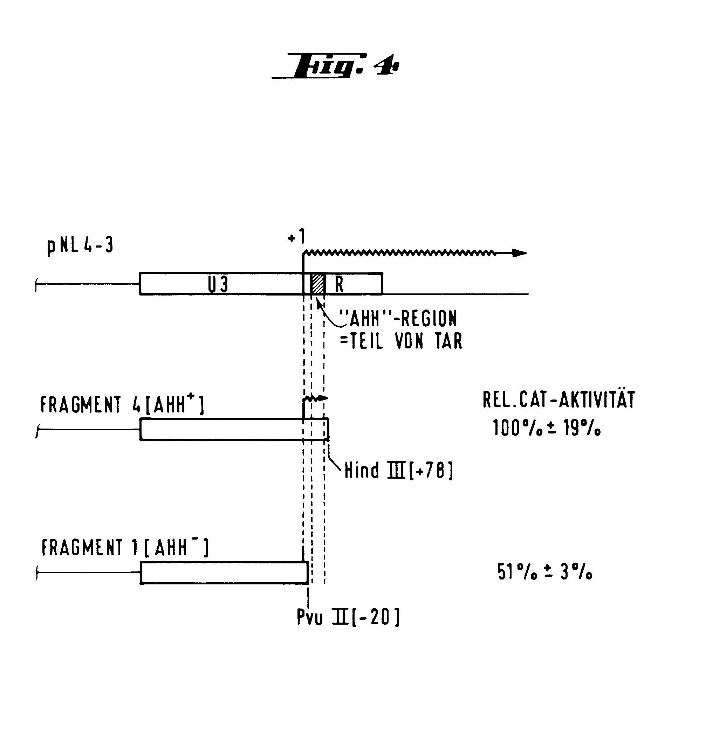

Fig. 4 beschreibt die Lage der AAH-Sequenz im rep-Gen.

Die biochemischen Eigenschaften und die biologische Rolle der rep-Proteine läßt vermuten, daß es zu einer Interaktion zwischen den rep-Proteinen und den HIV-1-spezifischen Nukleinsäuren kommt. Möglicherweise spielen dabei die DNA oder möglicherweise die RNA, die beide in der U3/R-Sequenz des HIV-1 enthalten sind, eine wichtige Rolle in dem Hemmungsmechanismus von HIV-1. Ein Vergleich der DNA-Sequenz zwischen der HAV-2 DNA und der HIV-1 LTR-Sequenz (Position 1-634, J. Virol. 59, S. 284-291 (1986)) zeigt einen 25 bp-Bereich mit 72 % Sequenzidentität bei den Positionen 16-40 auf der AAV-2 DNA und Positionen 483-507 auf der HIV-1 DNA, die Teil der tar-Sequenz ist.

Weiterhin ist ein Bereich mit einem hohen Grad an Homologie zwischen der errechneten lokalen Sekundärstruktur der HIV-1 tar-Region und der AAV-terminalen Sequenz zu finden. Dies zeigt, daß bei einer möglichen direkten oder indirekten Interaktion zwischen rep-Protein und HIV-1 RNA-Sequenz eine Sekundärstruktur-Erkennung beteiligt ist.

Das AAV rep-Gen hemmt die HIV-Replikation durch die Interaktion mit der Sequenz des HIV-1-LTR. Wie oben gezeigt, kann man durch Vergleich der Sequenzen von AAV-2 und der HIV-1-LTR-Region eine 25 bp lange Sequenz finden, wobei diese Region hochkonserviert ist. Diese Region wurde als "AHH" bezeichnet, wobei diese Region auch auf den Niveau der zweidimensionalen Struktur eine große Homologie zwischen AAV-2 und HIV-1 zeigt. Kompetitions-Experimente mit HIV-1-LTR Fragmenten zeigen, daß die Hemmung (in diesem Fall die rep-abhängige Hemmung der HIV-1-LTR getriebenen CAT-Expression) abhängig ist von der Anwensenheit der AAH-Region. Eine noch drastischere Aufhebung der rep-vermittelten Hemmung der HIV-1 LTR-getriebenen CAT-Expression konnte für ein 25 Basepaare langes, doppelsträngiges DNA-Fragment mit der AHH-Sequenz gezeigt werden. Dies zeigt eindeutig die Beteiligung der AHH-Region am Hemmechanismus.

Dieses Ergebnis zeigt in eindeutiger Weise das antivirale Potential der AAV-codierten rep-Gene. Der Umstand, daß das rep-Gen für die menschlichen SW480-Zellen untoxisch ist und daß durch die Anwesenheit des rep-Gens ein ausgesprochen hoher Hemmeffekt der HIV-Replikation zu beobachten ist, zeigt in sehr klarer Weise die hervorragenden Eigenschaften des Gegenstandes der vorliegenden Erfindung und die günstigen Voraussetzungen, die für eine Verwendung als antivirales Agens benötigt werden.

## Patentansprüche

1. Verwendung eines aus dem adeno-assoziierten Virus Typ 2 (AAV-2) stammenden rep-Gens oder einer Teilsequenz dieses Gens für die Herstellung eines spezifischen antiviralen Wirkstoffs zur Bekämpfung von Virusinfektionen.

2. Verwendung nach Anspruch 1, dadurch gekennzeichnet, daß das zu bekämpfende Virus das HIV-1 Virus ist.

3. Peptide, die Translationsprodukte des offenen Leserasters des AAV rep-Gens sind.

4. Peptide nach Anspruch 3, die Translationsprodukte der inserts der Plasmide pTAV2 oder pTAV2-6 sind.

5. Diagnostischer Kit enthaltend wenigstens ein Peptid der Peptide der Ansprüche 3 und 4.

## Patentansprüche für folgende Vertragsstaaten : ES, GR

1. Verwendung eines aus dem adeno-assoziierten Virus Typ 2 (AAV-2) stammenden rep-Gens oder einer Teilsequenz dieses Gens für die Herstellung eines spezifischen antiviralen Wirkstoffs zur Bekämpfung von Virusinfektionen.

2. Verwendung nach Anspruch 1, dadurch gekennzeichnet, daß das zu bekämpfende Virus das HIV-1 Virus ist.

3. Verfahren zur Herstellung von Peptiden mit antiviraler Aktivität, dadurch gekennzeichnet, daß der offene Leseraster des AAV rep-Gens oder Teile davon exprimiert wird.

4. Verfahren nach Anspruch 3, dadurch gekennzeichnet, daß die Plasmide pTAV 2 oder PTAV2-6 exprimiert werden.

5. Verfahren zum Nachweis von AAV-2, dadurch gekennzeichnet, daß mindestens ein Peptid, welches nach einem Verfahren nach Anspruch 3 oder 4 hergestellt wurde, verwendet wird.

| CO-MIKROINJIZIERTE PLASMIDE | AAV-MUTANT rep cap | | HIV-1 REPLIKATION [%] |
|---|---|---|---|
| pNL4-3 | | | 100.0 (28.0) |
| pNL4-3 + pTAV2 | + | + | 3.3 (1.0) |
| pNL4-3 + pTAV2-3 | − | + | 73.0 (16.0) |
| pNL4-3 + pTAV2-6 | + | − | 0.9 (0.5) |
| pNL4-3 + pTAV2-8 | − | − | 97.0 (29.0) |

0  20  40  60  80  100

**Fig. 1**

EP 0 486 917 A2

_Fig. 2_a

pTAV2-3    pTAV2-6    −    +

_Fig. 2_b

# Fig. 3

## FIG.3A)

pos. 483                   pos. 507

HIV-1    5'-CTGGGAGCTCTCTGGCTAACTAGGG-3'

AAV-2    5'-CTGCGCGCTCGCTCGCTCACTGAGG-3'

pos. 16                   pos. 40

## FIG.3B)

AHH-REGION

AAV-2 ssDNA
(pos.1-150)

AHH-REGION

HIV-1 RNA
(pos.1-58)

# Fig. 4

pNL 4-3

+1

U3    R

"AHH"-REGION
=TEIL VON TAR

FRAGMENT 4 [AHH$^+$]

REL.CAT-AKTIVITÄT
100% ± 19%

Hind III [+78]

FRAGMENT 1 [AHH$^-$]

51% ± 3%

Pvu II [-20]